# EUROPEAN PATENT SPECIFICATION

(11) **EP 1 017 354 B1**
(45) Date of publication and mention of the grant of the patent: **06.07.2005**
(21) Application number: 99937228.7
(22) Date of filing: 12.07.1999
(51) Int. Cl.: A61K 6/083

(54) **TRANSLUCENT WEAR RESISTANT DENTAL ENAMEL MATERIAL AND METHOD**
LICHTDURCHLÄSSIGES VERSCHLEISSFESTES ZAHNSCHMELZMATERIAL
EMAIL DENTAIRE TRANSLUCIDE RESISTANT A L'USURE, SES MATERIAUX CONSTITUTIFS ET PROCEDE ASSOCIE

(30) Priority: 20.07.1998 US 93364 P
(43) Date of publication of application: 12.07.2000
(73) Proprietor: Dentsply International, Inc., York, PA 17404-0872 (US)
(72) Inventor: WANG, Xiuling, Magnolia, DE 19962 (US); SUBELKA, John, C., Marlboro, NJ 07746 (US); JEFFERIES, Steven, R., York, PA 17402 (US); HAMMESFAHR, Paul, D., Wyoming, DE 19934 (US); SILVER, Paul, A., Wilmington, DE 19802 (US)
(74) Representative: Wächtershäuser, Günter, Prof. Dr.
(86) International application number: PCT/US1999/015602
(87) International publication number: WO 2000/003688

(56) References cited:
- EP-A- 0 475 239
- EP-A- 0 530 926
- EP-A- 0 717 976
- EP-A- 0 832 636
- EP-A- 0 839 511
- WO-A-98/43596
- US-A- 4 503 169
- US-A- 4 567 030
- PATENT ABSTRACTS OF JAPAN vol. 012, no. 482 (C-553), 15 December 1988 (1988-12-15) & JP 63 199204 A (TOKUYAMA SODA CO LTD), 17 August 1988 (1988-08-17)
- LEINFELDER: "An in vitro device for predicting clinical wear" QUINTESSENCE INTERNATIONAL, vol. 20, no. 10, 1989, pages 755-761, XP000856026 cited in the application

## Description

This is a continuation-in-part of U.S. Patent application Senal No.60/093,364 filed July 20, 1998 which is a continuation-in-part of U.S. Patent application Senal No. 09/136,320, filed July 6, 1998 (as case LDC-791 CON) which is a continuation-in-part of U.S Patent application Serial No 09/052,180 filed March 31, 1998, which is a continuation-in-part of patent application Serial No. 08/946,612 filed October 7, 1997, which have the benefit of the filing dates of provisional patent application 60/042,585 filed April 2, 1997; and provisional patent application Serial No. 60/043,812 filed Apnl 14, 1997.

The invention relates to a dental material useful in making artificial tooth enamel, inlays, onlays and veneers The invention provides dental material preferably having an opacity less than 50 percent and a localized wear volume loss of less than 0.025 mm³, formed from material having a hardenable matrix and a filler. A method according to the invention includes shaping the dental enamel material. Preferably the refractive index of the resin matrix material used to make artificial tooth enamel is within 5 percent of the refractive index of the filler material. More preferably, the refractive index of the resin matrix material is within 1 percent of the refractive index of the filler material. Most preferably, the refractive index of the resin matrix material is within 0.5 percent of the refractive index of the filler material.

The appearance of a dental restoration is modified not only by the intensity and shade of the pigments employed therein but also by the degree of translucency or opacity of the other material in the restorative. This is especially true of dental enamel.

### BACKGROUND OF THE INVENTION

In the dental enamel art, translucency (the inverse of opacity) is a characteristic which is essential and improvements of 5 percent or more are widely recognized as being significant improvements in the art particularly when accompanied by acceptable wear resistance.

It is greatly preferred that dental enamel material be effectively homogeneous such that air bubbles or structural discontinuities are substantially avoided from introduction into the tooth structure Additionally, it is preferred that such materials be capable of deforming a matrix band during the course of tooth filling. Such materials should also be capable of withstanding the physical stresses extant in the posterior region of the mouth and not crumble, fracture or erode under such conditions.

Opacity as used herein refers to the percentage of impinging white light transmitted from a spectrophotometer through a 1 mm thick sample of material being tested. More specifically, as used herein opacity of the sample of material, which is not pigmented, is measured using a Macbeth Color Eye Spectrophotometer calibrated according to the manufacturer's calibration method CAL-030-95, with the spectrophotometer connected to a CompuAid 286 microcomputer.

Localized wear volume loss (also known as volume loss of localized wear) as used herein refers to the volume loss in mm³ from a sample of material being tested after 250,000 cycles in a Leinfelder in vitro wear testing device as described in Lemfelder et al in An In Vitro Device for Predicting Clinical Wear, Quintessence International, Volume 20, Number 10/1989, pages 755-761. Measurements may be made for example using a VCA 2500 Video Contact Angle System, sold by AST Products, Inc., Billerica, MA, and a microcomputer with MicroSoft Windows software, in accordance with the AST products User's Manual. The wear pistons are calibrated with the return limit set to 8.3 mm, load set to 10Kg Maximum, (the load applied to the piston being from 7.6 to 8.0 Kg), Crosshead speed set to 200 mm/min. Such device is in use at University of Alabama, University of North Carolina, Creighton University and at DENTSPLY International Inc.

Localized extended wear volume loss as used herein refers to the volume loss in mm³ from a sample of material being tested after 400,000 cycles in a Leinfelder in vitro wear testing device as described in Leinfelder et al in An In Vitro Device for Predicting Climcal Wear, Quintessence International, Volume 20, Number 10/1989, pages 755-761. Measurements may be made for example using a VCA 2500 Video Contact Angle System, sold by AST Products, Inc., Billerica, MA, and a microcomputer with MicroSoft Windows software, in accordance with the AST products User's Manual. The wear pistons are calibrated with the return limit set to 8.3 mm, load set to 10Kg Maximum, (the load applied to the piston being from 7.6 to 8.0 Kg), Crosshead speed set to 200 mm/min. Such device is in use at University of Alabama, University of North Carolina, Creighton University and at DENTSPLY International Inc.

In measuring material volume loss the sample from the Lemfelder University of Alabama wear machine is measured for wear in the Form Taysurf profilometer which employs a transversing stylus to construct a 3D topographic map of the worn area by means of an electronic interface unit linked to a host computer. A surface analyzer program installed m the host computer graphically depicts the worn area and calculates its volume. This volume, expressed in cubic millimeters, is regarded as the "wear volume loss" of the material tested. The higher the volume loss, the greater the material wears.

The index of refraction (or refractive index) for any substance is the ratio of the velocity of light in a vacuum (Air at one atmospheric pressure is commonly used m place of a vacuum) to its velocity m the material being tested.

Liquid refractive index as used herein refers to the refractive index of a liquid. Preferably liquid refractive index as used herein is measured by a refractometer-Abbe Model (manufactured by BAUSH & LOMB).

Filler refractive index as used herein refers to the refractive index of inorganic filler particles. Preferably filler refractive index as used herein is that provided by the manufacturer, and may be measured, for example using a microscope.

Contrast ratio of dental composite is the ratio between the daylight apparent reflectance of the specimen when backed by a black standard and the reflectance of the specimen when backed by a white standard. The translucency of composite material is dependent on the particle size, shape and the difference in refractive indices between the glass filler and resin matrix in which the glass powders are located. The substantially perfect match (effective equality) in the refractive indices between inorganic glass filler and organic resin matrix used in the material of the invention results in the improved translucency of the dental enamel material of the invention.

Ethoxylated Bisphenol A Dimethacrylate (EBPADMA) also known as 2,2-Bis[4-(2-methacryloxyethoxy)phenyl]propane has the structural formula: 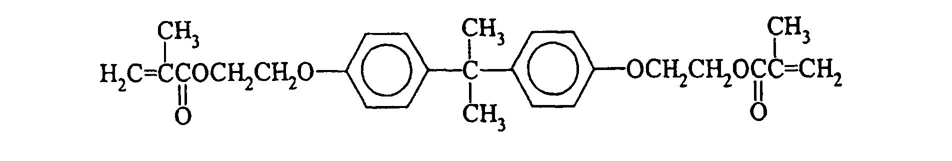 and is used as a resin matrix.

Triethylene glycol dimethacrylate also known as 2,2'[Ethanediylbis(oxy)bisethyl-di-2-methyl-propenate has the structural formula: 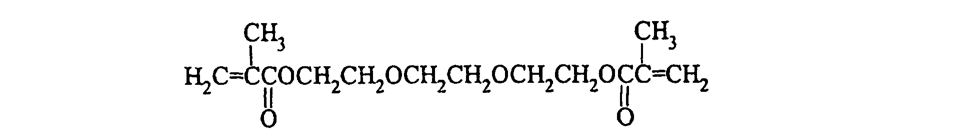 and is used as resin matrix.

Cyclodi-2,2'-bis{4-[3-methacryloxy-2-(1,12-dioxa-2,11-dioxo-3,10-diazadodecane)propoxy]phenyl} Propane (NCO Monomer) has the structural formula: 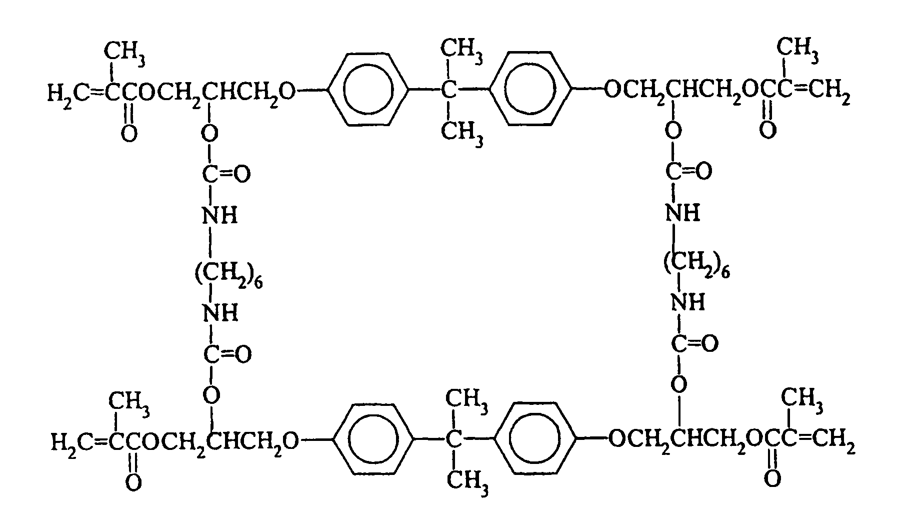 and is used as a resin matrix.

1,7,7,Trimethylbicyclo[2.2.1]heptane-2,3 dione (camphorquinone or CQ).

Ethyl-(4-N,N-dimethylamino)benzoate (EDAB) is a VLC initiating system and has a structural formula:

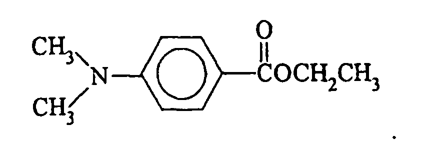

Butylated Hydroxy Toluene (BHT) also known as 2,6-bis(1,1-dimethylethyl)-4-methylphenol is a stabilizer having the structural formula:

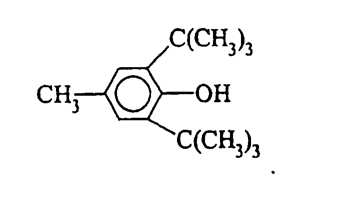

(2-Hydroxy-methoxyphenyl)phenyl Methanone is a UV stabilizer having the structural formula: 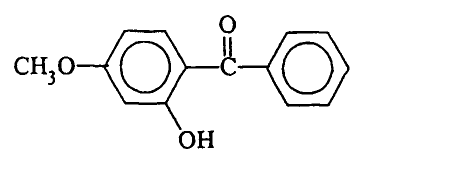 and sold by BASF Corporation as Uvinul M40.

Diethyl 2,5-dihydroxyterephthalate is a fluorescing agent having the structural formula: 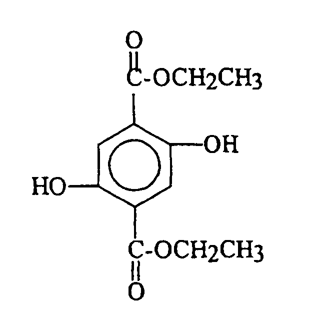 and is sold by Riedel-de Haen AG as Lumilux® Blau LZ.

2,7,7,9,15-Pentamethyl-4,13-dioxo-3,14-dioxa-5,12-diaza-hexadecane-1,16-diyldimethacrylate (UDMA) has a structural formula:

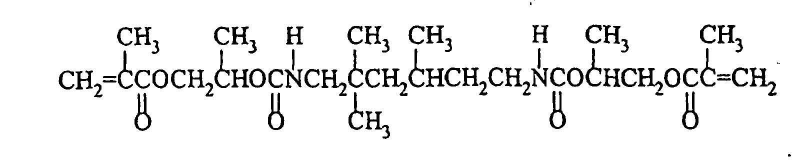

### THE OBJECTS OF THE INVENTION

It is an object of the present invention to provide a dental enamel material having an opacity less than 50 percent and a localized wear volume loss of less than 0.025 mm³.

It is a further object of the present invention to provide a polymerizable matrix forming liquid and inorganic filler particles, wherein the liquid comprises polymerizable material having a first refractive index, the filler comprises a plurality of particles having a second refractive index, and the first refractive index is within 5 percent of the second refractive index.

It is a further object of the present invention to provide such an enamel material, which is durable when, used in dental prosthetics.

These and other objects of the present invention which should become apparent from the description to follow, are carried out by the invention as hereinafter described and claimed.

### SUMMARY OF THE INVENTION

A method and material for forming translucent wear resistant dental enamel and the dental enamel material formed which has an opacity less than 50 percent and a localized wear volume loss of less than 0.025 mm³ and a localized extended wear volume loss of less than 0.04 mm³. The dental enamel material is formed from a polymerizable matrix and a filler. The material for forming translucent wear resistant dental enamel having an opacity less than 50 percent and a localized wear volume loss after 250,000 cycles of less than 0.025 mm³, comprises a polymenzable matrix forming liquid and inorganic filler particles. The liquid comprises polymenzable material having a first refractive index. The filler comprising a first plurality of particles having a second refractive index. The first plurality of particles being formed from a low and a high median particle size plurality of particles. The low median particle size plurality of particles having a median particle size between 0.3 and 0.7 micrometers. The high median particle size plurality of particles having a median particle size between 1 and 10 micrometers. The first refractive index being within 5 percent of said second refractive index. Said high median particle size plurality of particles have more preferably a median particle size between 1 and 2 micrometers. The filler also comprises a second plurality of filler particles having an average particle size of from about 0.01 to about 0.04 micrometers. Preferably the partcles of the second plurality of filler particles having an average particle size of from about 0.01 to about 0.04 micrometers are in agglomerated form having agglomerate sizes from about 0.2 micrometers to about 0.4 micrometers.

In accordance with a preferred embodiment of the invention is provided a material for forming translucent wear resistant dental enamel having an opacity less than 50 percent and a localized wear volume loss of less than 0.025 mm³, including a polymerizable matrix forming liquid and inorganic filler particles. The liquid compnses polymenzable material having a first refractive index. The filler comprises a first plurality of particles having a second refractive index. The first plurality of particles is formed from a low and a high median particle size plurality of particles. The low median particle size plurality of particles has a median particle size, between 0.3 and 0.7 micrometers. The high median particle size plurality of particles has a median particle size , between 1 and 10 micrometers and preferably between 1 and 2 micrometers. The first refractive index is within 5 percent of said second refractive index. The filler also comprises a plurality of filler particles having an average particle size of from about 0.01 to about 0.04 micrometers.

### PREFERRED EMBODIMENTS FOR CARRYING OUT THE INVENTION

A dental enamel material according to the present invention includes a hardenable resin and filler. The enamel material can be put in place with respect to the dentition to be restored and then sculpted or carved as needed due to its non-flowing characteristics. The enamel material of the invention is useful as an enamel coating over restroative material in a tooth in a patient's mouth, as a veneer, as an inlay and as an onlay. The dental enamel material includes a polymeric matrix and a filler component. The filler comprises a first plurality of particles having a second refractive index. The first plurality of particles is formed from a low and a high median particle size plurality of particles. The low median particle size plurality of particles has a median particle size, between 0.3 and 0.7 micrometers. The high median particle size plurality of particles has a median particle size , between 1 and 10 micrometers and preferably between 1 and 2 micrometers. The first refractive index is within 5 percent of said second refractive index. The filler also comprises a plurality of filler particles having an average particle size of from about 0.01 to about 0.04 micrometers.
One preferred filler material is a radiopaque dental glass. More preferably, the filler comprises a first plurality of preferably glass, more preferably barium glass, particles having an average particle size of from about 0.1 to about 10 and, a plurality of filler particles, preferably fumed silica, having an average particle size of from about 0.01 to about 0.04 micrometers. The inventive materials can be used as an intra-oral dental enamel, but can also be used by the laboratory technician in extra-oral dental applications such as in the production or restoration of crowns, inlays, and the like. The invention will be exemplified and discussed herein, for simplicity, with respect to intra-oral applications, it being understood that extra-oral applications are within the scope of the invention.

Examples of useful resins for compomers are those materials having as a principle functional ingredient, polymerizable unsaturated acidic monomers, such as a substituted butane moiety with acid or reactive acid derivative functionality. An example of an acid or reactive acid derivative functionality includes those having the general formula (RO₂C)ₓ-C₄H₆-(CO₂R')_{y} where R is an acid radical or reactive acid derivative and R' is a polymerizable unsaturated radical having from about 2 to about 13 carbon atoms, x is 2 to 3 and y is 1 to 2. A description of such materials is provided in U.S. Patent Number 5,218,070 which is herein incorporated by reference for such disclosure. A description of Eeamples of other useful resins is provided in U.S. Patent Number 5,338,773.

Any hardenable resin matrix useful in intra-oral or extra-oral dental applications is within the scope of the invention. Preferred resins include those that are curable, more preferably curable by exposure to actinic light. Examples of such resins include ethoxylated bisphenol-A-dimethacrylate; Bisphenol-A-Glycidylmethacrylate; tnethylene glycol dimethacrylate; and mixtures thereof. Optionally, a shading pigment or other additives may also be employed, such as for example, fluoride releasing agents, antibacterial agents, anticaries agents, and the like.

One preferred resin material is the reaction product of Bisphenol-A-Glycidylmethacrylate (Bis-GMA) and a chain initiator, such as hexamethylene diisocyanate (HMDI). The reaction may also include other reactive components. For example, the urethane component may be the reaction product of from about 27 to about 31 percent by weight of Bis-GMA as a reactive resin, more preferably about 29 percent by weight; from about 29 to about 33 percent by weight of triethylene glycol dimethacrylate (TEGDMA) as a reactive diluent, more preferably about 31 percent by weight; and, from about 29 to about 33 percent by weight of ethoxylated bisphenol-A-dimethacrylate (EBPADMA) also as a reactive diluent, more preferably about 31 percent by weight; with a useful amount of HMDI (preferably about 8 percent by weight). The reaction is preferably catalyzed with for example, a catalyst such as dibutyl tin dilaurate, and uses an inhibitor such as butylated hydroxy toluene.

Preferably, from about 97 to about 99 percent by weight of the urethane component, and more preferably about 98 percent by weight is used to form 100 percent by weight of the activated resin component. The remaining constituents of the activated resin include inhibitors, photoinitiators, UV absorbers, accelerators, fluorescing agents, and the like. While the preferred material is photocurable, a chemical cure package can also be used, including any of those well known in the art for dental use, including peroxide, amine, an ascorbic acid derivative, a metal ion salt, and the like.

Other useful resins can be employed including those disclosed in U.S. Patent Numbers 4,514,342; 4,675,941; 4,816,495; 5,338,773 and 5,710,194.

Examples of useful glass particles include barium aluminum-borosilicate glass, barium aluminofluorosilicate glass; mixtures thereof and the like. In these materials, barium can also be substituted by strontium and the like, and may also contain fluonde. Other useful materials include calcium hydroxyl ceramics, and others such as those fillers disclosed m U.S. Patent Numbers 5,338,773; 5,710,194; 4,758,612; 5,079,277 and 4,814,362. These materials may have any morphology or shape, including spheres, regular or irregular shapes, filaments or whiskers, and the like. Any particle shape having the other characteristics of the invention as described herein, including for example, average particle size, is within the scope of the invention. Preferred such glasses are also silanated although this is not an absolute limitation of the invention. The filler particles may be silane treated (silane coupled) or provided with other treatments as is conventional for dental fillers.

In addition to opacity improvements, the materials according to the invention when compared to dental composite materials previously known exhibit similar or improved physical characteristics. For example, these include depth of cure, diametral tensile strength, transverse rupture strength, flexural modulus, radiopacity, hardness, fracture toughness, polymerization shrinkage and wear. These characteristics and their comparisons with known compositions will be more fully explored hereinbelow. It has also been found that the present materials can be polished to a high luster with conventional polishing techniques. It will be shown that certain characteristics, especially opacity and wear resistance are improved over the prior art materials.

It has been found that compositions according to the invention have good or even improved aesthetic characteristics. The materials are polishable to a high luster despite being highly filled. It has also been found that the products have excellent radiopacity approaching that of gold and amalgam products. It has further been found that the inventive materials have equal or superior post-cure or polymerization shnnkage characteristics as compared to conventional materials. It is to be appreciated that the inventive material shows similar or improved physical characteristics compared to the commercially available product. Most notably, the inventive material shows an improved translucency, and opacity.

### EXAMPLE 1

A polymerizable monomeric resin matrix forming material having a refractive index of 1.52 was prepared by mixing at 23°C 49.33 parts EBPADMA urethane resin mixture (reactive methacrylate resin); 49.33 parts NCO monomer mixture (reactive methacrylate resin); 0.025 parts butylated hydroxy toluene (BHT, inhibitor); 0.2 parts diethyl-2,5-dihydroxy-terepthalate (fluorescing agent); 1.0 parts (2-hydroxy-4-methoxyphenyl)phenyl methanone (UV stabilizer); 0.10 parts camphorquinone (photo initiator); and 0.04 parts ethyl-(4-N,N-dimethylamino)benzoate(accelerator).

### EXAMPLE 1A

A polymerizable monomeric resin matrix forming material having a refractive index of 1.52 was prepared by mixing at 23°C 97.8 grams EBPADMA urethane resin mixture (reactive methacrylate resin); 0.025 grams butylated hydroxy toluene (BHT, inhibitor); 0.2 grams diethyl-2,5-dihydroxy-terepthalate (fluorescing agent); 1.0 grams (2-hydroxy-4-methoxyphenyl)phenyl methanone (UV stabilizer); 0.165 grams camphorquinone (photo initiator); and 0.81 grams ethyl-(4-N,N-dimethylamino)benzoate(EDAB) (accelerator).

### EXAMPLE 2

Filler blend was prepared by mixing barium fluoro alumino borosilicate glass (97 parts by weight) and silane treated fumed silica (TS720, 3.0 parts by weight). The barium fluoro alumino borosilicate glass has a refractive index of 1.52 and was first ground using a ball-mill to an average diameter of 1 0 µm and then the glass was silanated. The filler comprises a first plurality of glass particles having an average particle size of from about 1 to about 10 micrometers; a second plurality of glass particles having an average particle size of from about 0.1 to about 1 micrometers. The fumed silica comprises a plurality of filler particles having an average particle size of from about 0.01 to about 0.04 micrometers.

### EXAMPLE 3

Polymerizable composite forming composition was prepared by mixing 22.5 parts by weight of monomeric resin matrix (formed by following the procedure of Example 1) and 77.5 parts by weight of filler blend (prepared by following the procedure of Example 2). The polymerizable composition has the composition shown in Table 1. The composition is brushed onto an artificial tooth and polymerized to form a veneer composite having the physical properties shown m Table 1.

### EXAMPLE 4

Polymerizable monomeric resin matrix forming material having a refractive indexof 1.51 was prepared by mixing at 23°C 49.31 parts EBPADMA urethane resin (reactive methacrylate resin); 19.74 parts NCO monomer (reactive methacrylate resin); 29.61 parts UDMA (reactive methacrylate resin), 0.025 parts butylated hydroxy toluene (BHT, inhibitor); 0.2 parts diethyl-2,5-dihydroxy-terepthalate (fluorescing agent); 1.0 parts (2-hydroxy-4-methoxyphenyl)phenyl methanone (UV stabilizer); 0.10 parts camphorquinone (photo initiator); and 0 04 parts ethyl-(4-N,N-dimethylamino)benzoate(accelerator).

### EXAMPLE 5

Filler blend was prepared by mixing barium fluoro alumino borosilicate glass (99.5 parts by weight) and silane treated fumed silica (TS720, 0.5 parts by weight). The barium fluoro alumino borosilicate glass used has a refractive index of 1.52 and was first grounded using ball-mill to an average diameter of 10 µm and then the glass was silanated. The filler comprises a first plurality of glass particles having an average particle size of from about 1 to about 10 micrometers; a second plurality of glass particles having an average particle size of from about 0.1 to about 1 micrometers. The fumed silica comprises a plurality of filler particles having an average particle size of from about 0.01 to about 0.04 micrometers.

### EXAMPLE 6

Polymerizable composite forming composition is prepared by mixing 23 parts by weight of the polymerizable monomeric resin matrix (prepared by following the procedure of Example 4) and 77 parts by weight of filler blend (prepared by following the procedure of Example 5). The polymerizable composition has the composition shown in Table 1. The composition is brushed onto an artificial tooth and polymerized to form a composite veneer having the physical properties shown in Table 1.

**TABLE 1:**

| FORMULATION AND PHYSICAL PROPERTIES | | |
|---|---|---|
| EXAMPLE | 3 | 6 |
| | | |
| EBPADMA Urethane Resin | 11.10 | 11.34 |
| NCO Monomer | 11.10 | 4.54 |
| UDMA Resin | | 6.81 |
| BHT | 0.0058 | 0.0058 |
| Flublau Concentrate | 0.046 | 0.046 |
| Uvinul M40 | 0.23 | 0.23 |
| CQ | 0.023 | 0.023 |
| EDAB | 0.009 | 0.009 |
| Silanated Barium Alumino Fluorosilicate Glass (BAFG) | 75.18 | 76.61 |
| TS 720 | 2.32 | 0.39 |
| Compressive Strength (MPa) | 342.5 ± 54.4 | 392.8 ± 33.8 |
| Dimetral Tensile Strength (MPa) | 56.9 ± 6.3 | 56.2 ± 3.7 |
| Transverse Strength (MPa) | 143 ± 10 | 132 ± 21 |
| Flexural Modulus (GPa) | 9.6 ± 1.1 | 9.69 ± 0.62 |
| Barcol Hardness ("hard scale") | 77 | 72 |
| Depth of cure (mm) 20"/Spectrum | 13.2 | 9.94 |
| Water Sorption (µm/mm³) | 13.443 | 14.545 |
| Shrinkage (%) | 3.5345 | 3.1825 |
| Fracture Toughness (Mparn^{1/2}) | 1.53 ± 0.30 | 1 42 ± 0.12 |
| Localized wear volume loss (mm³) | 0.0123 | 0.0183 |
| Opacity (1 mm Thickness) | 45.92 | 36.03 |

Table 2 shows the localized wear volume loss from three commercial dental enamel products and the material formed in Example 3.

**TABLE 2**

| Product | Type | Manufacturer/ Owner | Volume Los (mm³) | Opaci |
|---|---|---|---|---|
| Example | Ename | DENTSPLY | 0.012 | 39.86 |
| Example | Ename | DENTSPLY | 0.0183 | 36.03 |
| Licupast | Ename | DeTech/DENTSPLY | 0.033 | 28.74 |
| Targis | Ename | Ivoclar | 0.043 | ---- |
| Artglass | Ename | Kulzer | 0.043 | 18.52 |
| Sculpture Super Cle | Ename | Generic/Pentron | ---- | 30.83 |

Wear resistant low opacity dental enamel composite materials of the invention are formed from mixtures of polymerizable liquid and filler particles. The polymenzable liquid has a liquid refractive index and filler has a filler refractive index. Low opacity dental enamel composite materials of the invention are formed from mixtures of polymerizable liquid having a liquid refractive index and filler particles having a filler refractive index which is about the same as the liquid refractive index. In order of increasing preference the filler refractive index is within 5 percent, 4 percent, 3 percent, 2 percent, 1 percent or 0.5 percent of the liquid refractive index. In accordance with a preferred embodiment of the invention at least 70 percent of the filler has a refractive index which is within, in order of increasing preference, 5 percent, 4 percent, 3 percent, 2 percent, 1 percent or 0 5 percent of the liquid refractive index. Preferably at least 80 percent of the filler has a refractive index which is within, in order of increasing preference, 5 percent, 4 percent, 3 percent, 2 percent, 1 percent or 0.5 percent of the liquid refractive index. More preferably at least 90 percent of the filler has a refractive index which is within, in order of increasing preference, 5 percent, 4 percent, 3 percent, 2 percent, 1 percent or 0.5 percent of the liquid refractive index.. Most preferably at least 99 percent of the filler has a refractive index which is within, in order of increasing preference, 5 percent, 4 percent, 3 percent, 2 percent, 1 percent or 0.5 percent of the liquid refractive index.

Preferably a material for forming translucent wear resistant dental enamel having an opacity less than 46 percent and a localized wear volume loss less than 0.025 mm³, comprising: a polymerizable matrix forming liquid and inorganic filler particles, the liquid comprising polymenzable material having a first refractive index, the filler comprising a first plurality of particles having a second refractive index and a second plurality of filler particles having an average particle size of from about 0.01 to about 0.04 micrometers, and the first refractive index being within 5 percent of the second refractive index.

Preferably the material comprises from about 12 to about 25 percent by weight of the polymerizable matrix forming liquid and from about 75 to about 88 percent by weight of the filler. Preferably the polymerizable matrix forming liquid comprises polymenzable unsaturated acidic monomers of a substituted butane moiety with an acid or reactive acid derivative functionality. Preferably the polymerizable matrix forming liquid comprises a photocurable resin. Preferably the opacity less than 46 percent and a localized wear volume loss less than 0.025 mm³. Preferably the first refractive index is within 3% of the second refractive index. Preferably the first plurality of filler particles comprises glass selected from the group consisting of barium aluminum-borosilicate; barium aluminofluorosilicate; strontium aluminum-borosilicate; strontium aluminofluorosilicate.

Preferably the second plurality of particles comprises fumed silica. Preferably the filler comprises from about 60 to about 90 of barium glass particles; and, from about 10 to about 30 of fumed silica particles Preferably the first refractive index is within 1 percent of the second refractive index. Preferably the second plurality of particles comprise less than 20 percent by weight of the filler and the first plurality of particles comprise more than 50 percent by weight of the filler. Preferably the second plurality of particles comprise less than 10 percent by weight of the filler and the first plurality of particles comprise more than 70 percent by weight of the filler.

The invention provides a method of forming translucent wear resistant dental enamel, comprising the steps of shaping polymerizable material to form tooth enamel; and, curing the polymenzable material; the polymenzable material comprising a polymenzable matrix forming liquid comprising polymerizable liquid having a first refractive index and filler particles; the filler comprising a first plurality of particles having a second refractive index and a second plurality of filler particles having an average particle size of from about 0.01 to about 0.04 micrometers, the first refractive index being within 1 percent of the second refractive index.

Preferably the invention provides a transparent wear resistant material having an opacity less than 46 percent and a localized wear volume loss less than 0.025 mm³, formed by the process comprising: providing a mixture comprising a polymerizable matrix forming liquid and filler particles; and cunng the liquid to form a dental enamel having an opacity of less than 50 percent and a localized wear volume loss of less than 0.025mm³. More preferably the invention provides dental enamel material having an opacity less than 46 percent and a localized wear volume loss less than 0.020 mm³, formed from material having a hardenable matrix and a filler which includes fillers having two different particle sizes. Most preferably transparent wear resistant material in accordance with the invention has an opacity less than 40 percent and a localized wear volume loss less than 0.019 mm³.

Preferably the liquid comprises polymenzable material having a first refractive index. Preferably the filler comprises a first plurality of particles having a second refractive index and an average particle size of from about 0.1 to about 10 micrometers and second plurality of filler particles having an average particle size of from about 0.01 to about 0.04 micrometers. Preferably the first refractive index is within 3 percent of the second refractive index. More preferably the first refractive index is within 1 percent of the second refractive index Most preferably the first refractive index is within 0.5 percent of the second refractive index.

In accordance with a preferred embodiment of the invention is provided a material for forming translucent wear resistant dental enamel having an opacity less than 50 percent and a localized wear volume loss less than 0.025 mm³, including a polymerizable matrix forming liquid and inorganic filler particles, The liquid comprising polymerizable material having a first refractive index. The filler comprising a first plurality of particles having a second refractive index. The first plurality of particles is formed from a low and a high median particle size plurality of particles. The low median particle size plurality of particles has a median particle size between 0.3 and 0.7 micrometers. The high median particle size plurality of particles has a median particle size between 1 and 2 micrometers. The first refractive index is within 5 percent of said second refractive index. Preferably, the material low median particle size plurality of particles comprises at least 40 percent by weight of the first plurality of particles. Preferably, the high median particle size plurality of particles comprises at least 40 percent by weight of the first plurality of particles. Preferably, the filler comprises more than 70 percent by weight of the material. Preferably, at least 90 percent by weight of the filler has a refractive index within 4 percent of the liquid refractive index and the filler further comprises a second plurality of filler particles having an average particle size of from about 0.01 to about 0 04 micrometers. Preferably, the liquid is cured to form polymeric material having a polymeric refractive index and at least 90 percent by weight of the filler has a refractive index within 2 percent of the polymeric refractive index. Preferably, at least 90 percent by weight of the filler has a refractive index within 1 percent of the liquid refractive index.

In each of Examples 7-9, 19.5 grams of the polymerizable monomeric resin matrix forming material formed by following the procedure of Example 1A is mixed with 80.5 grams of fillers to form polymerizable composite forming material. Table 3 shows the percent by weight of the fillers of Examples 7-9: course barium fluoride glass (mean particle size of 3.158 µm) and fine barium fluoride glass (average particle size of about 0.9 micron), 0X 50 glass and in Examples 8 and 9, Cab-O-Sil TS720 glass. 0 X 50 glass is fumed SiO₂, average particle size .04 microns sold by DeGussa. Cab-O-Sil TS 720 glass is fumed SiO₂, average particle size 0.01 microns sold by CABOT. In each of Examples 7-9, the polymerizable composite forming material is brushed onto a natural tooth in a patient's mouth to form a polymerizable dentin layer, which polymerizes upon curing by exposure for 10 seconds to light from a MaxLite light curing unit sold by DENTSPLY to form a polymeric dentin layer. Polymerizable composite forming composition prepared by following the procedure of Example 3 is brushed onto the polymeric dentin layer and cured by exposure to the light for 10 seconds to form a polymeric enamel layer.

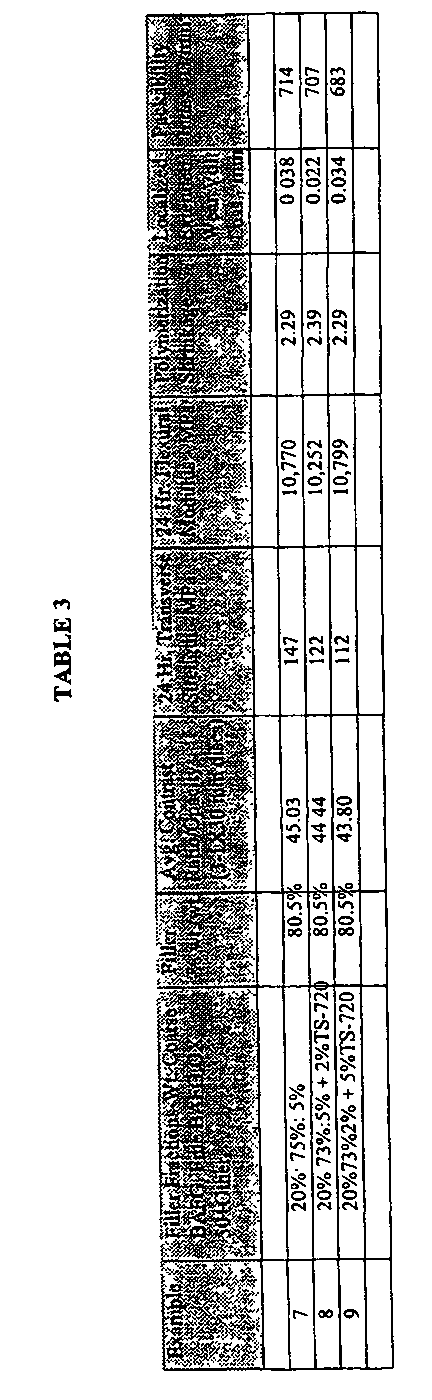

### EXAMPLE 10

23.0 grams of the polymerizable monomeric resin matrix forming material formed by the following the procedure of Example 1 is mixed with 77.0 gram of filler blend to form polymerizable composite forming material. The filler blend is formed by mixing 77.0 parts of fine milled barium fluoride glass, 20.0 parts of polymer coated silica (formed by following the procedure of Example 10A) and 3.0 parts Cab-O-Sil TS 720 fumed silica. 30 grams of polymerizable composite forming material is brushed onto a dental tooth in a patient's mouth and cured for 40 second of exposure to light from a Spectrum curing sold by DENTSPLY to form polymeric dentin layer of the tooth.

### EXAMPLE 10A

63.54 parts SiO₂ particles having average particle size of 0.04 microns, 22.22 parts bis-GMA, 13.65 parts TEGDMA monomers and 0.55 parts initiator are mixed together. Then the mixture is heated to form polymer containing silica. The polymer containing silica is gound to form polymer coated SiO₂ particle having an average particle size of 20 microns.

### EXAMPLE 11

Polymenzable composite forming composition was prepared by following the procedure of Example 3 is brushed onto the polymeric dentin layer of the tooth prepared by following the procedure of Example 10 and cured for 10 minutes of exposure to light from a Spectrum curing sold by DENTSPLY to form a veneer composite having the physical properties shown in Table 1 for Example 3.

### EXAMPLE 12

23.0 grams of the polymerizable monomeric resin matrix forming material formed by the following the procedure of Example 1 is mixed with 77.0 gram of filler blend to form polymerizable composite forming material. The filler blend is formed by mixing 77.0 parts of fine milled barium fluoride glass, 20.0 parts of polymer coated silica (formed by following the procedure of Example 10A) and 3.0 parts Cab-O-Sil TS 720 fumed silica. 30 grams of polymerizable composite forming material is brushed onto a shaped gold dental alloy substrate supported by a tooth model and cured for 40 second of exposure to light from a Spectrum curing sold by DENTSPLY to form polymeric dentin layer of an indirect restoration.

### EXAMPLE 13

Polymerizable composite forming composition was prepared by following the procedure of Example 3 is brushed onto the polymeric dentin layer of an indirect restoration prepared prepared by following the procedure of Example 12 and cured for 10 minutes of exposure to light from a Spectrum curing sold by DENTSPLY to form an indirect restoration having polymeric dentin layer and an enamel layer having the physical properties shown in Table 1 for Example 3

The foregoing description illustrates preferred embodiments of the invention. However, concepts employed may, based upon the description, be used in other embodiments without departing from scope of the invention.

## Claims

1. A material for forming translucent wear resistant dental enamel having an opacity less than 50 percent and a localized wear volume loss of less than 0.025 mm³, comprising:
(a) a polymerizable matrix forming liquid comprising polymerizable material having a first refractive index, and
(b) inorganic filler particles comprising
(b1) a first plurality of particles having a second refractive index, said first plurality of particles being formed from
(b1-1) a low median particle size plurality of particles having a median particle size between 0.3 and 0.7 micrometers,
(b1-2) a high median particle size plurality of particles having a median particle size between 1 and 10 micrometers,
and
(b-2) a second plurality of filler particles having an average particle size of from about 0.01 to about 0.04 micrometers,
whereby said first refractive index is within 5 percent of said second refractive index, and wherein said second plurality of filler comprises less than 10 percent by weight of said filler.

2. The material of claim 1 wherein said high median particle size plurality of particles have a median particle size between 1 and 2 micrometers.

3. The material of claim 1, wherein said first refractive index is within 3 percent, preferably 1 percent, of said second refractive index.

4. The material of any one of claims 1 or 2, wherein at least 90 percent by weight of said filler has said second refractive index within 4 percent, preferably 2 percent, more preferably 1 percent, and most preferably 0.5 percent, of said first refractive index of said liquid.

5. The material of any one of claims 1 or 2, wherein at least 99 percent by weight of said filler has said second refractive index within 4 percent, preferably 2 percent, more preferably 0.5 percent, of said first refractive index of said liquid.

6. The material of any one of claims 1 to 5, wherein said translucent wear resistant dental enamel has an opacity less than 46 percent and a localized wear volume loss less than 0.022 mm³.

7. The material of any one of claims 1 to 6, which comprises from about 12 to about 25 percent by weight of said polymerizable matrix forming liquid and from about 75 to about 88 percent by weight of said filler.

8. The material of any one of claims 1 to 7, wherein said polymerizable matrix forming liquid comprises polymerizable unsaturated acidic monomers of a substituted butane moiety with an acid or reactive acid derivative functionality.

9. The material of any one of claims 1 to 8, wherein said polymerizable matrix forming liquid comprises a photocurable resin.

10. The material of any one of claims 1 to 9, wherein said opacity is less than 46 percent, preferably 40 percent, and said localized wear volume loss is less than 0.022 mm³.

11. The material of any one of claims 1 to 10, wherein said first plurality of filler particles comprises glass selected from the group consisting of barium aluminum-borosilicate; barium aluminofluorosilicate; strontium aluminum-borosilicate; strontium aluminofluorosilicate.

12. The material of any one of claims 1 to 11, wherein said second plurality of particles comprises fumed silica.

13. The material of any one of claims 1 to 12, wherein said filler comprises from about 60 to about 90 percent by weight of barium glass particles; and from about 10 to about 30 percent by weight of fumed silica particles.

14. The material of claim 1, wherein said filler comprises less than 10 percent by weight of said second plurality of particles and more than 70 percent by weight of said first plurality of particles.

15. The material of any one of claims 1 to 14, wherein said first plurality of particles comprise at least 40 percent by weight of said low median plurality of particles.

16. The material of any one of claims 1 to 15, wherein said first plurality of particles comprises at least 40 percent by weight of said high median plurality of particles.

17. The material of any one of claims 1 to 16, which comprises more than 70 percent by weight of said filler.

18. A method of forming translucent wear resistant dental enamel, comprising the steps of shaping a material according to any one of claims 1 to 17 to form tooth enamel and curing said polymerizable material.

19. A transparent wear resistant material having an opacity less than 50 percent and a localized wear volume loss of less than 0.025 mm³, obtainable by the process of claim 18.

## Patentansprüche

1. Material zur Ausbildung eines transluzenten verschleißfesten Zahnschmelzes mit einer Opazität von weniger als 50% und einem lokalisierten Verschleißvolumenverlust von weniger als 0,025 mm³, das aufweist:
(a) eine polymerisierbare Matrix-bildende Flüssigkeit, die ein polymerisierbares Material mit einem ersten Brechungsindex aufweist, und
(b) anorganische Füllstoffteilchen, die aufweisen
(b1) eine erste Vielzahl von Teilchen mit einem zweiten Brechungsindex, wobei die erste Vielzahl von Teilchen gebildet wird aus
(b1-1) einer Vielzahl von Teilchen mit einer kleinen mittleren Teilchengröße, die eine mittlere Teilchengröße zwischen 0,3 und 0,7 µm aufweisen,
(b1-2) eine Vielzahl von Teilchen mit einer großen mittleren Teilchengröße, die eine mittlere Teilchengröße zwischen 1 und 10 µm aufweisen,
und
(b2)eine zweite Vielzahl von Füllstoffteilchen mit einer mittleren Teilchengröße von ca. 0,01 bis ca. 0,04 µm,
wobei der erste Brechungsindex nicht größer als 5% des zweiten Brechungsindex ist, und worin die zweite Vielzahl von Füllstoffteilchen weniger als 10 Gew.-% des Füllstoffes umfassen.

2. Material nach Anspruch 1, worin die Vielzahl von Teilchen mit einer großen mittleren Teilchengröße eine mittlere Teilchengröße zwischen 1 und 2 µm aufweisen.

3. Material nach Anspruch 1, worin der erste Brechungsindex nicht größer als 3%, vorzugsweise nicht größer als 1%, des zweiten Brechungsindex ist.

4. Material nach einem der Ansprüche 1 oder 2, worin mindestens 90 Gew.-% des Füllstoffes einen zweiten Brechungsindex aufweisen, der nicht größer als 4%, vorzugsweise nicht größer als 2%, insbesondere nicht größer als 1% und in erster Linie nicht größer als 0,5% des ersten Brechungsindex der Flüssigkeit ist.

5. Material nach einem der Ansprüche 1 oder 2, worin mindestens 99 Gew.-% des Füllstoffes einen zweiten Brechungsindex aufweisen, der nicht größer als 4%, vorzugsweise nicht größer als 2%, insbesondere nicht größer als 0,5%, des ersten Brechungsindex der Flüssigkeit ist.

6. Material nach einem der Ansprüche 1 bis 5, worin der transluzente verschleißfeste Zahnschmelz eine Opazität von weniger als 46% aufweist und einen lokalisierten Verschleißvolumenverlust von weniger als 0,022 mm³.

7. Material nach einem der Ansprüche 1 bis 6, das ca. 12 bis ca. 25 Gew.-% der eine polymerisierbare Matrix-bildenden Flüssigkeit und ca. 75 bis ca. 88 Gew.-% des Füllstoffes aufweist.

8. Material nach einem der Ansprüche 1 bis 7, worin die polymerisierbare eine Matrix bildende Flüssigkeit polymerisierbare ungesättigte saure Monomere einer substituierten Butan-Komponente mit einer Funktionalität einer Säure oder eines reaktiven Säurederivats aufweist.

9. Material nach einem der Ansprüche 1 bis 8, worin die polymerisierbare eine Matrix bildende Flüssigkeit ein photohärtbares Harz aufweist.

10. Material nach einem der Ansprüche 1 bis 9, worin die Opazität geringer als 46%, vorzugsweise geringer als 40%, ist, und der lokalisierte Verschleißvolumenverlust weniger als 0,022 mm³ beträgt.

11. Material nach einem der Ansprüche 1 bis 10, worin die erste Vielzahl von Füllstoffteilchen Glas umfasst, ausgewählt aus der Gruppe bestehend aus Bariumaluminiumborsilikat, Bariumaluminiumfluorsilikat, Strontiumaluminiumborsilikat, Strontiumaluminiumfluorsilikat.

12. Material nach einem der Ansprüche 1 bis 11, worin die zweite Vielzahl von Teilchen Quarzpulver umfasst.

13. Material nach einem der Ansprüche 1 bis 12, worin der Füllstoff ca. 60 bis ca. 90 Gew.-% an Barium-Glasteilchen und ca. 10 bis ca. 30 Gew.-% an Quarzstaub aufweist.

14. Material nach Anspruch 1, worin der Füllstoff weniger als 10 Gew.-% der zweiten Vielzahl von Teilchen und mehr als 70 Gew.-% der ersten Vielzahl von Teilchen umfasst.

15. Material nach einem der Ansprüche 1 bis 14, worin die erste Vielzahl von Teilchen mindestens 40 Gew.-% der Vielzahl von Teilchen mit einer kleinen mittleren Teilchengröße aufweist.

16. Material nach einem der Ansprüche 1 bis 15, worin die erste Vielzahl von Teilchen mindestens 40 Gew.-% der Vielzahl von Teilchen mit einer großen mittleren Teilchengröße aufweist.

17. Material nach einem der Ansprüche 1 bis 16, das mehr als 70 Gew.-% des Füllstoffs aufweist.

18. Verfahren zur Ausbildung eines transluzenten verschleißfesten Zahnschmelzmaterials, umfassend die Stufen des Gestaltens eines Materials nach einem der Ansprüche 1 bis 17 zur Ausbildung eines Zahnschmelzes und Härten des polymerisierbaren Materials.

19. Transparentes verschleißfestes Material mit einer Opazität von weniger als 50% und einem lokalisierten Verschleißvolumenverlust von weniger als 0,025 mm³, erhältlich nach dem Verfahren des Anspruchs 18.

## Revendications

1. Matière pour former un émail dentaire translucide, résistant à l'usure, ayant une opacité inférieure à 50 % et une perte de volume par usure localisée inférieure à 0,025 mm³, comprenant :
(a) un liquide polymérisable de formation de matrice, comprenant une matière polymérisable ayant un premier indice de réfraction, et
(b) des particules d'une charge inorganique comprenant :
(b1) une première pluralité de particules ayant un second indice de réfraction, ladite première pluralité de particules étant formée à partir
(b1-1) d'une pluralité de particules à petit diamètre médian de particules, ayant un diamètre médian de particules de 0,3 à 0,7 micromètre,
(b1-2) d'une pluralité de particules à grand diamètre médian de particules, ayant un diamètre médian de particules de 1 à 10 micromètres,
et
(b-2) une seconde pluralité de particules de charge ayant un diamètre moyen de particules d'environ 0.01 à environ 0,04 micromètre,
ledit premier indice de réfraction étant ainsi dans les limites de 5 % dudit second indice de réfraction, et ladite seconde pluralité de charge représentant moins de 10 % en poids de ladite charge.

2. Matière suivant la revendication 1, dans laquelle ladite pluralité de particules ayant un grand diamètre médian de particules a un diamètre médian de particules de 1 à 2 micromètres.

3. Matière suivant la revendication 1, dans laquelle ledit premier indice de réfraction est compris dans la limite de 3 %, de préférence de 1 %, dudit second indice de réfraction.

4. Matière suivant l'une quelconque des revendications 1 et 2, dans laquelle une proportion d'au moins 90 % en poids de ladite charge a ledit second indice de réfraction dans les limites de 4 %, avantageusement de 2 %, plus avantageusement de 1 % et de préférence de 0,5 %, dudit premier indice de réfraction dudit liquide.

5. Matière suivant l'une quelconque des revendications 1 et 2, dans laquelle une proportion d'au moins 99 % en poids de ladite charge a ledit second indice de réfraction dans les limites de 4 %, avantageusement de 2 %, plus avantageusement de 0,5 % dudit premier indice de réfraction dudit liquide.

6. Matière suivant l'une quelconque des revendications 1 à 5, dans laquelle ledit émail dentaire translucide, résistant à l'usure, a une opacité inférieure à 46 % et une perte de volume par usure localisée inférieure à 0,022 mm³.

7. Matière suivant l'une quelconque des revendications 1 à 6, qui comprend environ 12 à environ 25 % en poids dudit liquide polymérisable de formation de matrice et environ 75 à environ 88 % en poids de ladite charge.

8. Matière suivant l'une quelconque des revendications 1 à 7, dans laquelle ledit liquide polymérisable de formation de matrice comprend des monomères acides insaturés polymérisables d'un groupement butane substitué avec une fonctionnalité acide ou dérivée d'acide réactif.

9. Matière suivant l'une quelconque des revendications 1 à 8, dans laquelle ledit liquide polymérisable de formation de matrice comprend une résine photodurcissable.

10. Matière suivant l'une quelconque des revendications 1 à 9, dans laquelle ladite opacité est inférieure à 46 %, de préférence 40 %, et ladite perte de volume par usure localisée est inférieure à 0,022 mm³.

11. Matière suivant l'une quelconque des revendications 1 à 10, dans laquelle ladite première pluralité de particules de charge comprend du verre choisi dans le groupe consistant en borosilicate de baryum et d'aluminium ; aluminofluorosilicate de baryum ; borosilicate de strontium et d'aluminium ; aluminofluorosilicate de strontium.

12. Matière suivant l'une quelconque des revendications 1 à 11, dans laquelle ladite seconde pluralité de particules comprend de la silice ultrafine.

13. Matière suivant l'une quelconque des revendications 1 à 12, dans laquelle ladite charge comprend environ 60 à environ 90 % en poids de particules de verre au baryum ; et environ 10 à environ 30 % en poids de particules de silice ultrafine.

14. Matière suivant la revendication 1, dans laquelle ladite charge comprend moins de 10 % en poids de ladite seconde pluralité de particules et plus de 70 % en poids de ladite première pluralité de particules.

15. Matière suivant l'une quelconque des revendications 1 à 14, dans laquelle ladite première pluralité de particules comprend au moins 40 % en poids de ladite pluralité de particules à petit diamètre médian.

16. Matière suivant l'une quelconque des revendications 1 à 15, dans laquelle ladite première pluralité de particules comprend au moins 40 % en poids de ladite pluralité de particules à grand diamètre médian.

17. Matière suivant l'une quelconque des revendications 1 à 16, qui comprend plus de 70 % en poids de ladite charge.

18. Procédé pour former un émail dentaire translucide, résistant à l'usure, comprenant les étapes consistant à façonner une matière suivant l'une quelconque des revendications 1 à 17 pour former un émail dentaire et à faire durcir ladite matière polymérisable.

19. Matière transparente, résistant à l'usure, ayant une opacité inférieure à 50 % et une perte de volume par usure localisée inférieure à 0,025 mm³, pouvant être obtenue par le procédé de la revendication 18.
